# EUROPÄISCHE PATENTANMELDUNG

(11) **EP 2 671 612 A1**
(43) Veröffentlichungstag der Anmeldung: **11.12.2013**
(21) Anmeldenummer: 12171159.2
(22) Anmeldetag: 07.06.2012
(51) Int. Cl.: A61N 1/05, A61N 1/36, A61B 5/053

(54) **Verfahren zur Messung der Pharynxobstruktion mittels einer kombinierten Elektrode**

(71) Anmelder: Nasesch GbR, 10179 Berlin (DE)
(72) Erfinder: Seidl, Rainer Ottis, 10179 Berlin (DE)
(74) Vertreter: Lange, Sven

(57) **Zusammenfassung**

Die Erfindung beschreibt ein Verfahren für eine Beurteilung einer Dauer und eines Ausmaßes eines Pharynxverschlusses bei dem obstruktiven Schlafapnoesyndrom, wobei mittels mindestens einem Element Strom in eine Halsregion appliziert wird und eine Änderung einer Bioimpedanz durch mindestens ein Spannungsmesselement detektiert wird.

## Beschreibung

Die Erfindung betrifft ein Verfahren zur Messung der Pharynxobstruktion durch eine kombinierte Mess- und Stimulationselektrode, die vorzugsweise am Zungennerven angebracht wird und in der Lage ist die Position der Zunge im Rachen zu bestimmen und den Zungennerven so zu stimulieren, das er in eine vorbestimmte Position gebracht werden kann.

Bei obstruktiven schlafbezogenen Atmungsstörungen kommt es zu einem kompletten oder inkompletten Verschluss des Rachens während des Schlafs. Durch den Verschluss ist es dem Schlafenden nicht mehr möglich, zu atmen, dies führt mit der Zeit zu einem Abfall der Sauerstoffsättigung im Blut. Überschreitet die Anzahl und die Dauer der Phasen mit einer fehlenden Atmung in einer Nacht einen Grenzwert, so resultiert dies in vorübergehenden oder bleibenden Schäden z. B. an Herz, Lunge und Gehirn. Der Schlaf ist somit nicht mehr erholsam, sondern führt zu erheblichen Gesundheitsschäden.

Die Ursachen für ein obstruktives Schlafapnoe-Syndrom (OSAS) sind vielfältig. In den meisten Fällen kommt es durch ein Zurücksinken der Zunge und ein Erschlaffen des Rachens zu einem Verschluss oberhalb des Kehlkopfs. Da in einem gewissen Grad ein Aktivitätsverlust der Rachenmuskeln und Zungenmuskulatur während des Schlafs physiologisch ist, müssen in den meisten Fällen für das Vollbild eines OSAS weitere Faktoren vorhanden sein. Diese sind sowohl morphologische als auch koordinative Faktoren, wie z. B. anatomische Engen oder eine erhöhte Kollapsneigung der Muskulatur. Dabei kann sowohl jeder Faktor allein als auch eine gegenseitige Beeinflussung der Faktoren zu einer OSAS führen.

Standardtherapie des OSAS ist die kontinuierliche nasale Überdruckbeatmung (nasal Continuous Positive Airway Pressure, nCPAP). Hierbei wird über eine Nasenmaske ein positiver Luftstrom durch Mund und Nase in den Rachen gepresst, der als pneumatische Schienung wirkt und den Kollaps des Rachens verhindert. Das bedeutet für die Patienten, dass sie zur Nacht eine Beatmungsmaske tragen müssen und über ein Beatmungsgerät, das neben dem Bett steht, kontinuierlich beatmet werden. Die Anwendungsdauer dieser Beatmung ist in der Regel lebenslang. Die Akzeptanz für diese Behandlungsmethode ist eingeschränkt (65-80%). Patienten beklagen die Lärmentwicklung durch das Beatmungsgerät und den für Lebenspartner abschreckenden Anblick eines Menschen mit einer Atemmaske an einem Beatmungsgerät. In vielen Fällen müssen die Patienten alleine schlafen (H. Becker u. a., "Long-term acceptance of nasal continuous positive airway pressure therapy in 70 patients with sleep apnea over a 6-month treatment period", Pneumologie (Stuttgart, Germany), Bd. 43 Suppl 1, S. 643-646, Nov. 1989).

Es wurde neben der Beatmung noch eine Vielzahl weiterer Therapiemethoden erprobt (z. B. Protrusionsschienen, die den Unterkiefer vorschieben, um den Rachen zu öffnen, sowie eine Vielzahl von verschiedenen Operationen), die bisher in Studien keine positiven Ergebnisse gezeigt haben.

Im Verlauf der letzten 10 Jahre wurden mehrfach Versuche unternommen, durch eine muskuläre oder nervale elektrische Stimulation das Zurücksinken der Zunge zu verhindern und somit den Verschluss des Rachens im Schlaf zu verhindern. Zu Beginn der Forschungen erfolgte die elektrische Stimulation über transkutan-perkutane und intraoral platzierte Elektroden. Stimuliert werden sollten die Muskeln, die zu einem Herausstrecken der Zunge führen (z.B. M. genioglossus). Hierbei traten allerdings methodische Probleme auf. Zum einen konnte der M. genioglossus nicht ausreichend selektiv stimuliert werden, zum anderen kam es zusätzlich zur Aktivierung weiterer Muskelgruppen, die nicht gewünscht war. Diese unselektive Stimulation bewirkte zusammengenommen sogar eine Verengung des Durchmessers im Rachen. Zusätzlich blieb die Stimulation der Muskulatur im Schlaf durch die Patienten nicht unbemerkt, es kam zu Weckreaktionen (Arousal) bei den Patienten. Später gelang es durch die Anwendung von Elektroden, die durch den Mund in Muskeln gelegt wurden, selektiv geeignete Muskelgruppen zu stimulieren. Durch niedrigere Stimulationsschwellenwerte wurde die Zahl der Weckreaktionen reduziert.

In tierexperimentellen Untersuchungen konnte gezeigt werden, dass es durch elektrische Stimulation eines distalen Ausläufers des N. hypoglossus zu einem Herausstrecken der Zunge und damit zu einer Öffnung des Rachens kommt. Diese selektive Nervstimulation führte zu einer Abnahme des Rachenkollaps und einer Verbesserung der Menge eingeatmeter Luft. Die Einstellung der Stimulation erfolgt dabei am wachen Patienten. Dabei wird über eine Modifikation der Spannung, Impulsdauer und Frequenz solange eine "Titration" des elektrischen Stimulus durchgeführt, bis es ohne Angabe subjektiver Beschwerden zu einem Herausstrecken der Zunge kommt.

Diese Ergebnisse waren Grundlage für die Entwicklung einer Reihe von Implantaten, die in verschiedenen Zulassungsstudien geprüft wurden oder noch geprüft werden. Die Prinzipien der Stimulation entsprechen dabei den bisher vorgestellten. Durch die elektrische Stimulation kommt es zu einer Vorwärtsbewegung der Zunge und damit zu einer Erweiterung des Rachens. Die Einstellung der Stimulation erfolgt am wachen Patienten. Die Implantate stimulieren dabei nur eine Zungenseite. Stimuliert wird bei jedem Einatmen des Patienten. Die Atemtätigkeit wird dabei z. B. durch Drucksensoren, die in den Brustraum implantiert werden, ermittelt. Außerdem kann z. B. die Atmungserkennung mittels einer implantierten Bioimpedanzmessung am Brustraum erfolgen. Alle Implantate werden zur Nacht durch den Patienten eingeschaltet und nach dem Schlaf wieder ausgeschaltet. Zugängliche Zwischenauswertungen der bisher vorliegenden Studienergebnisse zu den Implantaten zeigen, dass es durch die einseitige Stimulation des distalen N. hypoglossus zu einer Öffnung des Rachens beim Einatmen und zu einer Besserung der Schlafphasen und der Sauerstoffsättigung kommt. Diese Verbesserungen sind jedoch lageabhängig; In Rückenlage reicht die Stimulation nicht aus, um den Rachen ausreichend zu öffnen.

Experimentelle Untersuchungen sowohl an Tieren als auch am Menschen demonstrieren, dass durch eine allgemeine Tonuserhöhung der Zungenmuskulatur eine bessere Öffnung der Atemwege mit einer geringeren Weckreaktionen durch die Stimulation erzielt werden kann als bei den bisherigen Implantaten. Dies erfordert jedoch eine selektive Stimulation des proximalen N. hypoglossus, um gezielt die innen- und außenliegenden Muskelgruppen der Zunge zu aktivieren. Dies ist notwendig, um durch eine gleichzeitige Aktivierung antagonistischer Muskeln eine Tonuserhöhung herbeizuführen. Die Machbarkeit einer selektive Stimulation des proximalen N. hypoglossus mittels spezieller mehrkanaliger Cuff-Elektroden wurde zunächst in Tierexperimenten nachgewiesen. Seit kurzem führt die Firma ImThera Medical, Inc. Zulassungsstudien mit einem neuen humanen Implantat durch, das eine 6-kanalige Cuff-Elektrode verwendet. Bei dem System wird durch selektive Stimulation des proximalen N. hypoglossus der Muskeltonus der Zunge erhöht. Da dauerhaft während des Schlafs stimuliert wird, ist bei diesem System keine Sensorik zur Erkennung der Atemtätigkeit erforderlich. Für eine Tonuserhöhung ist eine geringere Reizintensität notwendig, sodass die Patienten durch die Stimulation weniger belästigt werden. Dadurch soll die Anzahl der Weckreaktionen vermindert werden. Weitere Ergebnisse zu der Methode liegen noch nicht vor.

Die Stimulation der Zungennerven erfolgt derzeit durch Elektroden, die um den Nerv gelegt werden. Häufig handelt es sich dabei um sogenannte Cuff-Elektroden oder Cuffelektroden, ähnlich wie sie auch für die Stimulation und Ableitung des peripheren Nervensystems genutzt werden. Diesen Ansatz verwendet das Inspire-System der Firma Medtronic, wobei die Cuff-Elektrode den Nerv nicht vollständig umschließt, sondern lediglich als "halber Cuff" ausgebildet ist. Die Inspire-Elektrode ist durch ein unter der Haut geführtes Kabel mit einem implantierbaren Stimulator verbunden, der sich wie ein Herzschrittmacher unterhalb des Schlüsselbeins befindet.

Aufgrund seiner guten mechanischen und biologischen Eigenschaften wird in den meisten Fällen Silikon als Basismaterial für Elektroden eingesetzt. In die Silikonmanschette werden Metallstrukturen eingearbeitet, sodass im Inneren der Manschette mehrere Elektroden Kontakt mit der Nervoberfläche haben. Diese Kontakte werden meist aus Platin gefertigt, da dieses Material hervorragende Eigenschaften für die Elektrostimulation (gute Korrosionsbeständigkeit, hohe reversibel übertragbare Ladungsdichten) aufweist. In der Regel wird für Cuff-Elektroden eine Konfiguration von drei ringförmigen Elektroden gewählt, sodass bei der Stimulation die beiden äußeren Ringe als Gegenelektrode verwendet werden können. Hierdurch kann das elektrische Feld auf einen definierten Bereich fokussiert und Streuströme reduziert werden. Dieser Effekt wird zusätzlich durch die isolierende Wirkung der Silikonmanschette verstärkt.

Alternativ bietet sich der Einsatz von Polyimid an. Mit diesem Werkstoff lassen sich folienartige Elektroden mit sehr geringer Dicke (10 µm) generieren, in die über Fotolithographieprozesse strukturierte Metallschichten als Leiterbahnen und Elektrodenkontakte eingebaut werden können. Im Vergleich zu Silikonelektroden bietet die Polyimidtechnologie die Möglichkeit, exakte Strukturen mit einer höheren Auflösung zu erzeugen.

Obwohl das Konzept und die ersten Ergebnisse einer elektrischen Stimulation in der Therapie des OSAS erfolgversprechend waren, bestehen noch erhebliche, vor allem technische Probleme. Dies ist vor allem auf folgende Punkte zurückzuführen: Am schwerwiegendsten ist, dass bei den bisher vorgestellten Implantaten keine Erfolgskontrolle der Stimulation stattfindet. Die Stimulationssysteme werden am wachen Patienten eingestellt. Ziel der Einstellung ist eine maximale Öffnung des Rachens durch ein Herausstrecken oder eine Tonuserhöhung der Zunge. Eine weitere Anpassung der Stimulation während des Schlafs und eine Erfolgskontrolle finden nicht statt. Die Stimulation erfolgt nicht in Abhängigkeit vom Ausmaß des Pharynxverschlusses, sondern getriggert durch die Atemtätigkeit oder permanent im Fall der Tonuserhöhung. Ursache für dieses Problem ist das Fehlen eines einfachen, implantierbaren Messsystems, das in der Lage ist, den Verschluss des Rachens während des Schlafs sicher zu erfassen.

Außerdem sind für die bisher vorliegenden einkanaligen Stimulationssysteme Sensoren notwendig, die die Atemtätigkeit erfassen. Diese Systeme müssen am Brustkorb befestigt werden, z. B. in Höhe des Zwerchfells oder durch das Brustbein. Das erfordert einen nicht unerheblichen operativen Aufwand, da entweder ein Loch durch das Brustbein gebohrt werden muss, was das Risiko von Infektionen trägt oder es müssen Kabel am gesamten Brustkorb unter der Haut bis zum Zwerchfell geführt werden. In den bisher vorliegenden Arbeiten zum Inspire I wurde von nicht unerheblichen technischen Schwierigkeiten durch die Sensoren berichtet, so dass die Hälfte der Implantate nach einem Zeitraum von 6 Monaten defekt waren.

Dementsprechend war es Aufgabe der Erfindung ein Verfahren und ein System bereitzustellen, was nicht die Nachteile oder Mängel des Standes der Technik aufweist und Dauer und Ausmaß des Pharynxverschlusses bei dem obstruktiven Schlafapnoesyndrom bestimmt.

Gelöst wird die Aufgabe durch die unabhängigen Ansprüche. Vorteilhafte Ausführungsformen finden sich in den Unteransprüchen.

Es war völlig überraschend, dass ein Verfahren für eine Beurteilung einer Dauer und eines Ausmaßes eines Pharynxverschlusses bei dem obstruktiven Schlafapnoesyndrom bereitgestellt werden konnte, das nicht die Nachteile und Mängel des Standes der Technik, wobei mittels mindestens einem Element Strom in eine Halsregion appliziert wird und eine Änderung einer Bioimpedanz durch mindestens ein Spannungsmesselement detektiert wird. Das Spannungsmesselement detektiert bevorzugt einen Verschluss der Atemwege während des Schlafens detektiert, wobei vorteilhafterweise die Änderung der Bioimpedanz während einer Annäherung von Kehlkopf, Kehldeckel, Zungengrund an eine Rachenhinterwand gemessen wird. Es war völlig überraschend, dass ein Verfahren zur Messung der Pharynxobstruktion durch eine kombinierte Mess- und Stimulationselektrode bereitgestellt werden kann, wobei die kombinierte Elektrode vorzugsweise am Zungennerven angebracht wird und in der Lage ist die Position der Zunge im Rachen zu bestimmen und den Zungennerven so zu stimulieren, das er in eine vorbestimmte Position gebracht werden kann.

Dies bedeutet eine wegweisende technische Vereinfachung und methodische Erweiterung der Diagnostik und Therapie bei einem Schlafapnoe. Dies gilt nicht nur im Rahmen eines Implantats sondern auch für die Erfolgskontrolle einer Therapie, wie z.B. der CPAP-Beatmung.

Es hat sich bei der Entwicklung des Messverfahrens gezeigt, dass das entwickelte Messsystem ein erhebliches Marktpotential hat, da zur Zeit keine alternativen Messverfahren bekannt sind und das Messverfahren einen schnellen Eingang in den klinischen Alltag finden kann. Mit einer Erweiterung der diagnostischen Möglichkeiten entsteht eine erhöhte diagnostische Sicherheit in der Therapie des Schlafapnoe. So steht zu erwarten, dass die Indikationsstellung für eine Vielzahl von operativen Maßnahmen, die im Rahmen der Therapie des OSAS zum Einsatz kommen, eine Grundlage erhält. Damit wird das bevorzugte Messverfahren in Zukunft Voraussetzung für die Planung weiterer Therapiemaßnahmen sein.

Die Messung der Bioimpedanz ist dem Fachmann zur Bestimmung des Flüssigkeitshaushalts von Menschen und anderen Lebewesen bekannt. Die Messung der Bioimpedanz kann zudem auch für die Messung des Körperfettanteils genutzt werden. Bei der Messung der Bioimpedanz wird mit einem schwachen Wechselstrom über eine, vorzugsweise zwei Elektroden ein elektromagnetisches Feld im Körper aufgebaut. Über eine, vorzugsweise zwei weitere Elektroden im Inneren dieses Feldes wird dann der Spannungsabfall und die Phasenverschiebung der Signalspannung gemessen (Vierleitermessung). Die Teilwiderstände Resistanz und Reaktanz (die Induktion hat keine Bedeutung) sind abhängig von der Länge, vom Volumen und den intra- und extrazellulären Verteilungsräumen der Körperflüssigkeiten sowie von den Kapazitäten der Zellmembranen. Hierdurch können z. B. Änderungen der Körperflüssigkeiten in den Verteilungsräumen gemessen werden. Es gibt insbesondere zwei mögliche Verfahren um Bioimpedanz (erfindungsgemäß auch als BI bezeichnet) zu messen. Bei der 2-Elektroden-Methode wird die Spannung direkt über die Stromelektroden gemessen. Dabei wird der Spannungsabfall über dem Elektroden-Haut-Kontakt mit gemessen. Der Spannungsabfall entsteht durch den Stromfluss, der über die Stromelektroden in den Patienten eingeprägt wird. Dieser Widerstand ist zeitvariant und führt damit zu einem Messfehler. Ein solch unerwünschter Effekt kann durch die 4-Elektroden-Methode vermieden werden. Hierbei wird die Spannung über zusätzliche Elektroden gemessen. Da ein vernachlässigender Strom durch die Spannungselektroden fließt, kommt es auch zu keinem störenden zeitvarianten Spannungsabfall aufgrund des Elektroden-Haut-Kontakts. Die bevorzugte Ausführungsform des Verfahrens unterstützt beide Methoden.

Es war völlig überraschend, dass die Dauer und eines Ausmaßes des Pharynxverschlusses bei dem obstruktiven Schlafapnoesyndrom mittels der Messung der Bioimpedanz erfolgen kann. Die einfache Handhabung ermöglicht einen Einsatz des Verfahrens, der nicht von Fachpersonal vorgenommen oder betreut werden muss. Dadurch verringern sich die Kosten für die Anwendungen erheblich. Ein weiterer Vorteil der Erfindung ist, dass die Messungen immer und sofort erfolgen können. Außerdem muss die Person, an der das Verfahren appliziert wird, keine aufwendige und vor allem unangenehme Atemmaske tragen. Die detektierte Bioimpedanzänderung kann mit Kontrollwerten verglichen werden. So können beispielsweise auch eigene Fortschritte beurteilt werden.

Die Änderung der Bioimpedanz wird vorzugsweise während der Annäherung von Kehlkopfs und Zungenbein bestimmt. Es war völlig überraschend, dass die Annäherung von Kehlkopf und Zungenbein zu einer mit dem verfahren messbaren Änderung der Bioimpedanz im Hals führt. Dies unterscheidet die Erfindung von bisher bekannten BI-Messverfahren. Somit kann Verschluss der Atemwege durch die Annäherung von Kehlkopf und Zungenbein mit dem erfindungsgemäßen Verfahren schnell und eindeutig beurteilt werden.

Es ist bevorzugt, dass das Element zum Applizieren eines Stroms eine Elektrode ist. Die Elektrode ist bevorzugt eine Cuffelektrode (ebenfalls als Cuff-Elektrode bezeichnet). Das Prinzip, dass Muskelkontraktionen über elektrische Stimulationen ausgelöst werden können, ist schon mehr als 200 Jahre lang bekannt. Stand der Technik für implantierbare Elektroden und Leitungen ist die Verwendung von Platin, Platin- Iridium oder Stainless Steel 316als Leiter und Silikonkautschuk PTFE, PU oder PE als Isolator. Die als Wendel verarbeiteten Litzendrähte gewährleisten mechanische Flexibilität und Stabilität. Alle bisher klinisch eingesetzten Elektroden stimulieren nervale Strukturen und liegen entweder am Epineurium, am Motorpoint oder am Muskel. Der Entwicklungsstand klinisch implantierbarer Stimulatoren liegt zur Zeit bei induktiv versorgten Viel- Kanal-Implantaten (bis zu 21 Kanäle) mit einer extrakorporalen Einheit zur Energieversorgung und Steuerung.

Die Elektronik kann vorzugsweise durch ein Metall- oder Keramikgehäuse hermetisch geschützt werden, wobei das Ganze oder ein Teil des Gehäuses, die Antenne und die Elektrodenstecker z. B. in Hysol(Epoxi)- oder Silastic (Silikonkautschuk) eingebettet werden. Neuere Generationen von Implantaten, und zwar Microcontroller-gesteuerte Vielkanal-Implantate mit integrierter Batterieversorgung und induktiv versorgte Implantate für denervierte Muskulatur, haben einen vorklinischen Entwicklungsstand erreicht.

Die Leitermaterialien der bevorzugten Elektroden sind insbesondere Platin, Platiniridium oder Stainless Steel 316 in verschiedenen Kombinationen und verschiedener Verarbeitung. Bei diesen Materialien sind mechanische Robustheit und Korrosionsbeständigkeit sowie Biokompatibilität kontrollierbar. Beispielsweise lässt sich für Stainless Steel 316 eine Ladungsgrenze von 50 µC/cm2 pro Impuls definieren, die eine Reversibilität der elektrolytischen Prozesse an der Elektrodenoberfläche und damit eine Langzeitbeständigkeit und Langzeitbiokompatibilität gewährleistet. Als Isolationsmaterialien sind Polymere bevorzugt. Polymere bezeichnen im Sinne der Erfindung insbesondere eine Substanz, die sich aus einem Kollektiv chemisch einheitlich aufgebauter, sich in der Regel aber hinsichtlich Polymerisationsgrad, Molmasse und Kettenlänge unterscheidender Makromoleküle (Polymermoleküle) zusammensetzt. Bei solchen polymereinheitlichen Stoffen sind alle Makromoleküle bevorzugt gleich aufgebaut und unterscheiden sich lediglich durch ihre Kettenlänge (Polymerisationsgrad). Man kann derartige Polymere als Polymerhomologe bezeichnen. Polymere können aus der Gruppe umfassend anorganische Polymere, metallorganische Polymere voll- oder teilaromatischen Polymeren, Homopolymere, Copolymere, Biopolymere, chemisch modifizierte Polymere und/oder synthetische Polymere ausgewählt werden und umfassen Polyethylen, Polypropylen, Polyvinylchlorid, Polystyrol, Polymethylmethacrylat, Polyamid, Polyester, Polycarbonat, Polyethylenterephthalat, Polyethylenglykol, Dendrimere, Silikone oder Polyhydroxxyalkanoate. Bevorzugt wird die Elektrode aus Silikon, bevorzugt Silikonkautschuk, Polytetrafluorethen, Polyurethan oder Polyethylen hergestellt. Silikone bezeichnen insbesondere eine umfangreiche Gruppe von synthetischen polymeren Verbindungen, in denen Silicium-Atome über Sauerstoff-Atome kettenartig und/oder netzartig verknüpft und die restlichen Valenzen des Siliciums durch Kohlenwasserstoff-Reste (meist Methyl-Gruppen, seltener Ethyl-Gruppen, Propyl-Gruppen, Phenyl-Gruppen u. a.) abgesättigt sind.

Es ist bevorzugt, dass die Elektrode eine Cuffelektrode ist, also eine, einen Nerv umschließende Elektrode. Dieser Elektrodentyp ist im Wesentlichen nicht mehr in Verwendung, da es zu zahlreichen Zwischenfällen mit traumatischen Nervläsionen bei Zug an der Elektrode oder durch Kompression wegen Bindegewebsbildung zwischen Elektrode und Nerv gekommen ist. Es war dementsprechend überraschend, dass mittels der bevorzugten Cuffelektrode eine Applizierung von Strom und/oder Messung einer Spannung erfolgen kann, ohne dass es zu Schädigungen des Nerves kommt.

Es kann bevorzugt sein, die Elektrode und die Leitungen aus Platin oder Platiniridium anzufertigen, wobei die Leitungen in ein Polymer ein anderes Isolationsmaterial eingebettet sind. Die Elektrode verfügt vorzugsweise über einen Kontakt, über den Strom übertragen und/oder die Spannung gemessen wird.

In einer bevorzugten Ausführungsform des Verfahrens wird mittels mindestens zwei räumlich getrennter Elektroden mit jeweils einem Kontakt, ein Strom appliziert und gleichzeitig die Spannung gemessen. Die Elektroden können insbesondere als Implantat aber auch subkutan vorliegen. Weiterhin ist es bevorzugt, dass mittels mindestens zwei räumlich getrennter Elektroden ein Strom appliziert und die Spannung gemessen wird, wobei die Elektroden jeweils mindestens zwei Kontakte aufweisen und über einen Kontakt der Strom appliziert und über einen anderen Kontakt die Spannung gemessen wird. Die Elektroden können als Implantat vorliegen oder transkutan appliziert sein.

Des Weiteren ist bevorzugt, dass mindestens vier Elektroden mit jeweils einem Kontakt vorliegen, wobei über zwei Elektroden Strom appliziert wird und über zwei Elektroden die Spannung gemessen wird. Die Elektroden können transkutan realisiert sein oder als Implantat vorliegen.

Insbesondere bevorzugt ist, wenn die Elektroden als mehrkanalige Cuffelektroden vorliegen und auf beiden Seiten am Nervus hypoglossus befestigt sind, wobei auf der Außenseite jeder Cuffelektrode mindestens ein Kontakt zur Applizierung des Stroms und Messung der Spannung vorhanden ist. Diese Positionen haben sich als besonders vorteilhaft erwiesen, da besonders genaue Messwerte erzielt werden konnten. Die Cuffelektrode kann bevorzugt sowohl Längs- als auch Binnenmuskulatur der Zunge für eine Erhöhung des Muskeltonus selektiv stimulieren.

Es ist bevorzugt, dass die Elektroden beidseitig auf dem Musculus sternocleidomastoideus in Höhe des Unterkiefers und/oder auf dem Schildknorpel in Höhe oder unterhalb oder oberhalb der Stimmbandebene angeordnet sind. Die hat sich als vorteilhaft erwiesen, da es für unterschiedlichen Zungen- und Rachenanatomien angewendet werden kann.

In einer weiteren besonders bevorzugten Ausführungsform werden die Elektroden, jeweils umfassend ein Spannungsmesselement und ein Element zum Applizieren eines Stroms, beidseitig vor dem Musculus sternocleidomastoideus zwischen Zungenbein und Schildknorpel oder beidseitig auf dem Schildknorpel in Höhe oder unterhalb oder oberhalb der Stimmbandebene angeordnet. Es hat sich herausgestellt, dass mittels dieser Anordnung kleinste Abweichungen der Bioimpedanz von Normwerten messbar sind, so dass ein Positionsverlagerung der Zunge frühzeitig feststellbar und korrigierbar ist.

Weiterhin kann es bevorzugt sein, die Elektroden beidseitig in Höhe Zungenbein und Schildknorpel vor dem Musculus sternocleidomastoideus angeordnet sind. Es hat sich gezeigt, dass die Änderung der Bioimpedanz während der Annäherung von Kehlkopf und hinterem Zungenbein besonders gut detektiert werden kann, wenn die Spannungsmesselektroden beidseitig zwischen Zungenbein und Schildknorpel vor dem Musculus sternocleidomastoideus angeordnet sind. Die hier genannten Positionen sind besonders geeignet, um exakte Messwerte zu erhalten, da große Amplituden erzielt werden können. Außerdem werden mit den genannten Messanordnungen keine falsch-positiven oder falsch-negativen Ergebnisse generiert. Die Positionen eignen sich außerdem gut für die genannten Anwendungen, da die Elektroden in unkomplizierter Weise an die entsprechenden Stellen angebracht werden können und durch Schlucken oder Atembewegungen nicht verrutschen. Der Fachmann versteht die technische Lehre der vorliegenden Erfindung dahingehend, dass je nach Geschlecht, Größe, Gewicht und Alter, die Anatomie der Patienten eine leichte Veränderung der Positionen der Elektroden erfordern kann. Der Fachmann ist in der Lage die Elektroden je nach den anatomischen Voraussetzungen des einzelnen Patienten so anzubringen, dass eine optimale Messung möglich ist.

Die Erfindung betrifft weiterhin ein Schlafapnoeerkennungssystem zur Durchführung des Verfahrens zur Diagnose eines Pharynxverschlusses bei dem obstruktiven Schlafapnoesyndrom, umfassend mindestens ein Elektrode zur Applizierung eines Stroms und/oder zur Messung einer Spannung. Die Merkmale des oben dargestellten Verfahrens sind analog für das bevorzugte System anzuwenden. Das Schlafapnoeerkennungssystem umfasst bevorzugt zwei räumlich voneinander getrennte Elektroden zur Applizierung eines Stroms und/oder zur Messung einer Spannung. Eigene Untersuchungen haben gezeigt, dass ein Messsystem mit einem eigenentwickelten und innovativen Bioimpedanz-Messsystem vorliegt. Dabei zeigte sich völlig überraschend, das mit dem Bioimpedanzsystem der zunehmende Verschluss des Rachens durch ein Erschlaffen der Zungen und Pharynxmuskulatur erfassen ließ. Vergleichende endoskopische Untersuchungen bei Patienten das mit einem nachgewiesenen OSAS zeigten, dass mit einer kutanen BI-Messung eine Beurteilung des Verschlussgrades des Rachens sicher möglich ist.

In einer bevorzugten Ausführungsform ist eine Stromquelle mit einer Frequenz von 25 kHz bis 200 kHz, bevorzugt 50 kHz oder 100 kHz mit mindestens einer Elektrode verbunden. Es kann aber auch bevorzugt sein, zwei Stromquellen mit mindestens zwei Elektroden zu verbinden, die sich im Frequenzbereich unterscheiden.

Zudem ist ein Schlafapnoeerkennungssystem bevorzugt, wobei das System mindestens einen Bandpassfilter zur Entfernung von Störartefakten und Isolierung einer Messfrequenz umfasst. Bevorzugt wird der Bandpassfilter nach dem Messverstärker eingesetzt. Störartefakte auf den Spannungsmesselementen, die durch Muskelaktionspotentiale und Kabelbewegungen verursacht werden können, lassen sich leicht mittels des Bandpasses entfernen, da die BI-Änderungen in der amplitudenmodulierten Spannung in einem engen Frequenzbereich um die gewählte Messfrequenz enthalten sind. Ferner ist das System robuster gegenüber externen Störungen und weist eine höhere zeitliche Auflösung auf als beispielsweise die Elektrotomographie und mehrkanalige Elektroglottographie.

In einer bevorzugten Ausführungsform ist die Stromquelle eine differentielle Stromquelle, die eine schwebende Last symmetrisch ansteuert, Gleichtaktstörungen minimiert, bevorzugt unterdrückt, im Wesentlichen keinen Gleichstromanteil zulässt und robust gegenüber Erdungen der Last gegen Masse ist. Diese Ausführung ist vorteilhaft, da sie Gleichströme bei der Messung und Messfehler durch Berührungen des Patienten im Wesentlichen verhindert und robust gegenüber Erdungen der Last gegen Masse ist. Besonders bevorzugt wird eine Stromquelle gemäß DE 601 25 601 T2 verwendet. Die DE 601 25 601 T2 ist in den Offenbarungsgehalt dieser Erfindung mit aufgenommen. Außerdem bevorzugt ist die Verwendung einer Gleichstrombarriere zum Patienten. Bevorzugt wird die Gleichstrombarriere an beide Ausgänge der Stromquelle eingesetzt. Bevorzugt wird die Gleichstrombarriere nach den Dioden zum Überspannungsschutz eingesetzt. In der bevorzugten Ausführungsform werden als Gleichstrombarriere zum Patienten Kondensatoren, vorzugsweise Y1-Kondensatoren, eingesetzt. Weiterhin bevorzugt ist eine komplette galvanische Isolierung jeder einzelnen differentiellen Stromquelle. Die schaltungstechnische Realisierung der BI-Messung vereinfacht sich, da nur der sich ändernde Betrag der BI bestimmt werden muss. Dessen Verlauf kann aus der Einhüllenden der gemessenen Spannung gewonnen werden.

Durch die Regelung von zwei Stromflüssen über beide Elektroden können einige Probleme aus dem Stand der Technik verhindert werden. Das Berühren des Patienten, während der Messung, führt zwar auch zu einem Stromfluss über den Therapeuten, der aber durch Nachregelung des Stromflusses ausgeglichen wird, sodass eine große Fehlerquelle ausgeschaltet werden konnte.

Weiterhin betrifft die Erfindung eine Cuffelektrode zur Messung der Bioimpedanz und Stimulation der Zunge, wobei die Elektrode an einer Innen- und Außenseite Elektrodenkontakte aufweist. Die Cuffelektrode besteht vorzugsweise aus Polyimid oder Silikon. Zudem sind die oben aufgeführten Materialien bevorzugt. Poyimide bezeichnen im Sinne der Erfindung insbesondere Polymere, deren Wiederholungseinheiten durch Imid-Gruppen zusammengehalten werden. Die Imid-Gruppen können als lineare oder cyclische Einheiten vorliegen. Zu den Polyimiden werden insbesondere auch Polymere gerechnet, die neben Imid- auch Amid- (Polyamidimide), Ester- (Polyesterimide) und Ether-Gruppen (Polyetherimide) als Bestandteile der Hauptkette enthalten. Im Unterschied zu den bereits bestehenden Systemen werden die Cuff-Elektroden Kontakte sowohl an der Innen- als auch an der Außenseite aufweisen. Dies ermöglicht die simultane Messung der Bioimpedanz und Stimulation des N. hypoglossus mit einem integrierten System. Eine bevorzugt mehrkanalige Cuff-Elektrode für eine selektive, beidseitige, Stimulation des N. hypoglossus, die gleichzeitig in der Lage ist, die Bioimpedanz zu erfassen, ist nicht im Stand der Technik beschrieben. Die günstige anatomische Lage des N. hypoglossus nach der Unterkreuzung der A. carotis externa an der Pharynxwand ermöglicht es, Mess- und Stimulationselektroden (oder Kontakte) auf eine beidseitig zu verwendende Cuff-Elektrode zu vereinen. Dabei soll die Innenseite der mehrkanaligen Cuff-Elektrode eine selektive Stimulation von Nervenbündeln ermöglichen, während über die außenliegenden Elektroden eine Erfassung der Bioimpedanz zwischen den beidseitig platzierten Cuff-Elektroden realisiert werden kann. Dies würde den Aufwand bei einer operativen Implantation erheblich vermindern und damit die Risiken und Komplikationen für den Patienten. Durch eine mehrkanalige Stimulation soll eine differenzierte Stimulation der Zunge möglich werden, die z.B. auch auf Positionsänderungen des Kopfes reagieren kann.

In einer weiteren Aspekt betrifft die Erfindung ein Implantat, umfassend ein Schlafapnoeerkennungssystem, wobei das Implantat kontinuierlich und quantitativ einen Rachenverschluss erfasst und durch eine geregelte und selektive Stimulation des Nervus hypoglossus den Tonus der Zunge erhöht. Der Einsatz einer Neuroprothese für die Behandlung von Schlafapnoe ist von hervorragender klinischer Bedeutung. Der Rehabilitation von Schlafapnoe wird dadurch eine neue, zukunftweisende Perspektive eröffnet. Bis zum heutigen Zeitpunkt stehen nur wenige valide Therapieoptionen der Behandlung von Schlafapnoe zur Verfügung. Die bekannten Therapien sind eine erhebliche Belastung für Patienten, Angehörige und Kostenträger. Durch die unterstützenden Maßnahmen einer geregelten Stimulation im Rahmen einer Neuroprothese gelingt es diesen Verlauf zu verändern und zu verkürzen, was für die Patienten eine deutliche Verminderung ihrer Behinderungen, eine Verbesserung ihrer Lebensqualität und eine erhebliche Einsparung für die Kostenträger bedeutet.

Die verwendeten Ausdrücke haben in dem betreffenden Fachgebiet eine allgemein anerkannte Bedeutung, sodass der Fachmann in der Lage ist, das dargestellte Verfahren, System, Elektrode oder Implantat umzusetzen.

Die Erfindung soll im Folgenden anhand von Beispielen und Figuren erläutert werden, ohne jedoch hierauf beschränkt zu sein. Es zeigen:
- Fig. 1: Messkurve der Pharynxobstruktion mit Bioimpedanz
- Fig. 2: Aufnahmen des Larynxeingangs
- Fig. 3: Mess- und Stimulationselektrode
- Fig. 4: Intraoperatives Bild
- Fig. 5: Schemazeichnung eines bevorzugten Implantates

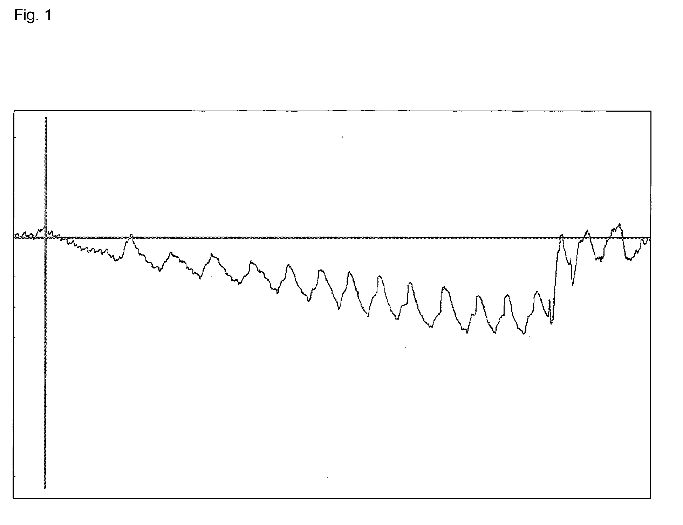

Fig. 1 zeigt eine Messung der Pharynxobstruktion mit Bioimpedanz (Bioimpedanzkurve während einer Apnoephase (ca. 2 min und 20 s)). Beispielhafte Aufzeichnung der Bioimpedanz während einer Videoschlafendoskopie bei einem Patienten mit einer nachgewiesenen Schlafapnoe mit ausgeprägten Sättigungsabfällen (die Versuche wurden bei einer Vielzahl von Patienten durchgeführt und werden hier nur beispielhaft wiedergegeben): In Zusammenschau mit den endoskopischen Bildern ist erkennbar, wie der Kehlkopf an die Pharynxrückseite sinkt und bei den Versuchen Einzuatmen an der Rachenhinterwand angesaugt wird. Dabei kommt es zu einem kontinuierlichen Absinken der Bioimpedanz. Gelingt es dem Patienten wieder Atem zu holen, kehrt die Bioimpedanz auf ihren Ausgangswert zurück. Dieser Fall zeigt, dass bereits vergleichsweise geringe Einengungen des Larynxeingangs, die nicht in einem vollständigen Zurücksinken des Zungengrundes an die Rachenhinterwand begründet sind, mit der Bioimpedanz abgebildet werden können.

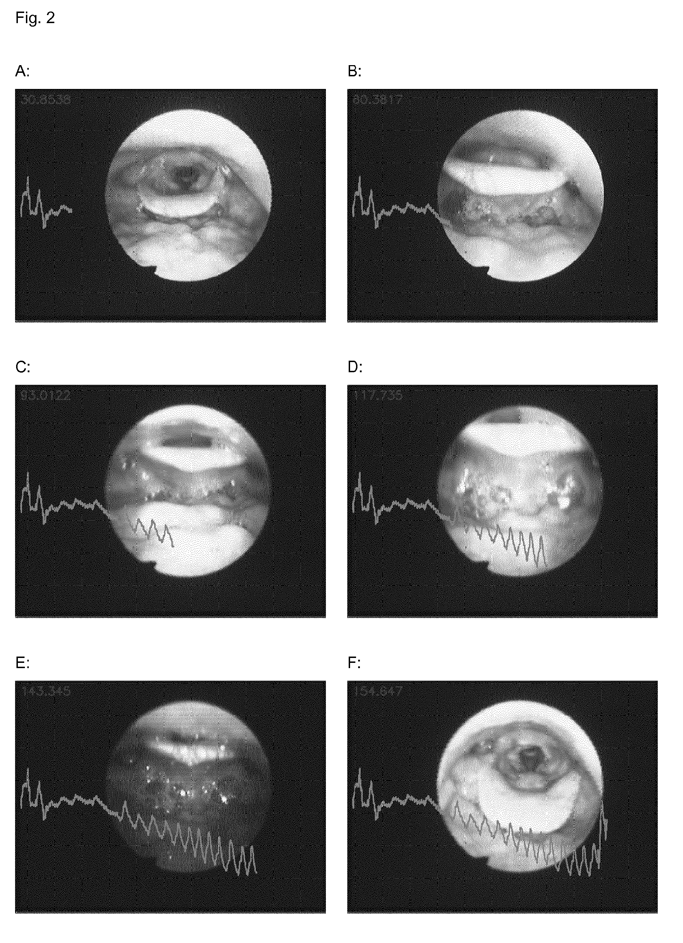

Fig. 2: Aufnahmen des Larynxeingangs.
A: Ausgangpunkt mit einem weit geöffneten Larynxeingang während der Einatmung: Es ist ein weiter Blick in die Trachea möglich.
B: Der Kehlkopf nähert sich der Pharynxrückseite an, die Stimmbänder sind geschlossen.
C: Weitere Annäherung des Kehlkopfes an die Pharynxrückseite; Gleichzeitig verkleinern sich die Sinus piriformis neben dem Kehlkopf, dies zeigt sich in der Bioimpedanz mit einem weiteren Abfall der Messkurve. Während des Einatmens kommt es zu einem Absinken der Bioimpedanz, da der Zungengrund durch den Unterdruck aus dem Thorax an die Rachenhinterwand angesogen wird.
D: Die Epiglottis liegt nun nahezu an der Rachenhinterwand an. Die Sinus piriformis sind vollständig geschlossen. Die Bioimpedanz ist weiter abgefallen. Die Intervalle, in denen der Patient versucht einzuatmen, werden kürzer.
E: Die Epiglottis liegt der Pharynxrückseite an sowie Anteile des Zungengrundes. Die Einatemintervalle verkürzen sich weiter, während die Tiefe der thorakalen Exkursionen zunimmt, wie an der Bioimpedanzkurve ablesbar ist.
F: Mit der Öffnung des Larynx kehrt die Bioimpedanzkurve wieder auf ihren Ausgangswert zurück.

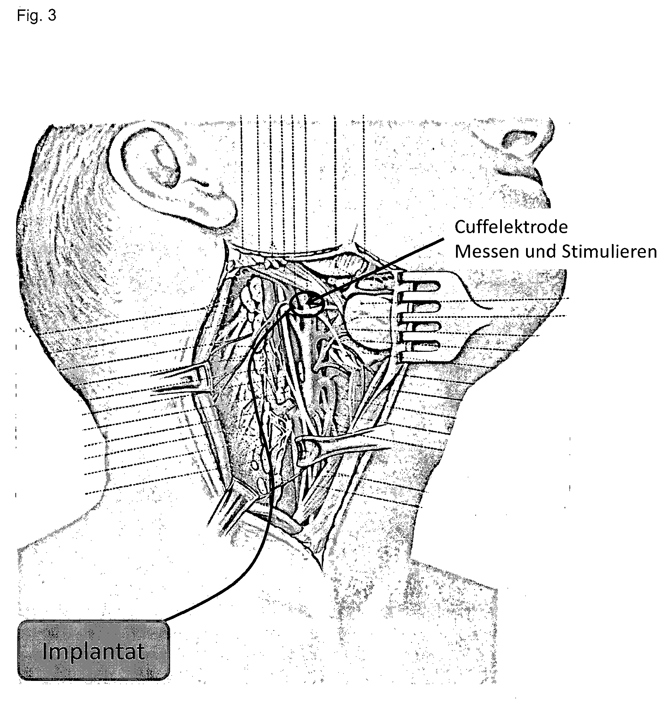

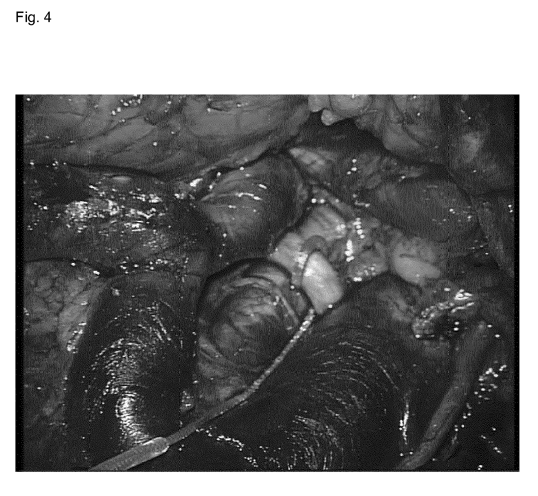

Fig. 3 zeigt eine bevorzugte Mess- und Stimulationselektrode und Fig. 4 ein intraoperatives Bild. Schemazeichnung der Elektrodenposition und intraoperative Bild (Fig. 4) wie die bevorzugte Cuff-Elektrode dem Nerven anliegt.

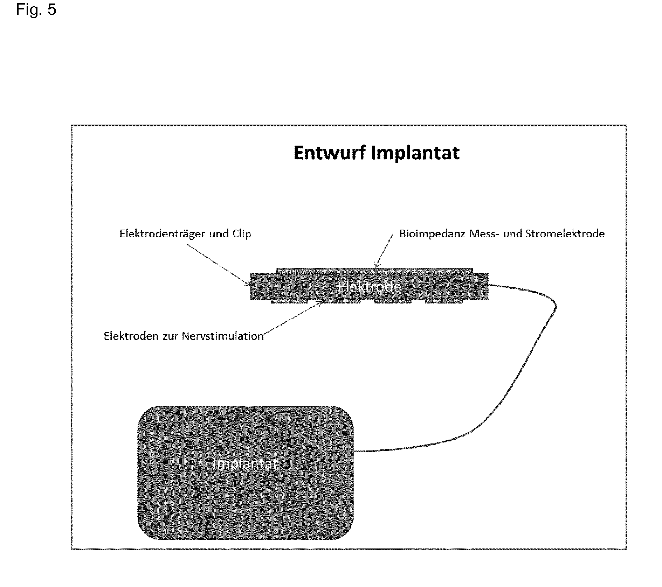

Fig. 5 Schemazeichnung eines bevorzugten Implantates. Dargestellt ist eine kombinierte Mess- und Stimulationselektrode, wobei die Außenseite wird vorteilhafterweise für die Messung und die Innenseite für die Stimulation genutzt wird. Die bevorzugte Elektrode weist einen Elektrodenträger mit einem Clip auf, auf dem die Mess- und Stromelektrode und vorzugsweise die Elektroden zur Nervenstimulation aufgebracht sind.

## Patentansprüche

1. Verfahren für eine Beurteilung einer Dauer und eines Ausmaßes eines Pharynxverschlusses bei dem obstruktiven Schlafapnoesyndrom, wobei mittels mindestens einem Element Strom in eine Halsregion appliziert wird und eine Änderung einer Bioimpedanz durch mindestens ein Spannungsmesselement detektiert wird.

2. Verfahren insbesondere nach Anspruch 1 zur Messung der Pharynxobstruktion durch eine kombinierte Mess- und Stimulationselektrode bereitgestellt werden kann, wobei die kombinierte Elektrode vorzugsweise am Zungennerven angebracht wird und in der Lage ist die Position der Zunge im Rachen zu bestimmen und den Zungennerven so zu stimulieren, das er in eine vorbestimmte Position gebracht werden kann.

3. Verfahren nach Anspruch 1 oder 2, wobei das Spannungsmesselement einen Verschluss der Atemwege während des Schlafens detektiert.

4. Verfahren nach einem oder mehreren der vorherigen Ansprüche, wobei die Änderung der Bioimpedanz während einer Annäherung von Kehlkopf, Kehldeckel, Zungengrund an eine Rachenhinterwand gemessen wird.

5. Verfahren nach einem oder mehreren der vorherigen Ansprüche, wobei das Element zum Applizieren eines Stroms eine Elektrode ist und/oder die Elektrode das Spannungsmesselement aufweist und/oder über einen Kontakt verfügt, über den Strom übertragen und/oder die Spannung gemessen wird.

6. Verfahren nach einem oder mehreren der vorherigen Ansprüche, wobei mindestens zwei räumlich getrennte Elektroden mit jeweils einem Kontakt verwendet werden, über die der Strom appliziert und die Spannung gemessen wird oder mindestens zwei räumlich getrennte Elektroden mit jeweils mindestens zwei Kontakten genutzt werden, wobei über einen Kontakt der Strom appliziert und über einen anderen Kontakt die Spannung gemessen wird.

7. Verfahren nach einem oder mehreren der vorherigen Ansprüche, wobei mindestens vier Elektroden mit jeweils einem Kontakt genutzt werden und über zwei Elektroden Strom appliziert und über zwei Elektroden die Spannung gemessen wird.

8. Verfahren nach einem oder mehreren der vorherigen Ansprüche, wobei die Elektrode oder die Elektroden transkutan oder als Implantat vorliegen.

9. Verfahren nach einem oder mehreren der vorherigen Ansprüche, wobei die Elektroden als mehrkanalige Cuffelektroden vorliegen und auf beiden Seiten am Nervus hypoglossus befestigt sind, wobei auf der Außenseite jeder Cuffelektrode mindestens ein Kontakt zur Applizierung des Stroms und Messung der Spannung vorhanden ist.

10. Verfahren nach einem oder mehreren der vorherigen Ansprüche, wobei die Cuffelektrode sowohl Längs- als auch Binnenmuskulatur der Zunge für eine Erhöhung des Muskeltonus selektiv stimuliert.

11. Verfahren nach einem oder mehreren der vorherigen Ansprüche, wobei die Elektroden beidseitig auf dem Musculus sternocleidomastoideus in Höhe des Unterkiefers und/oder auf dem Schildknorpel in Höhe oder unterhalb oder oberhalb der Stimmbandebene angeordnet sind oder die Elektroden beidseitig in Höhe Zungenbein und Schildknorpel vor dem Musculus sternocleidomastoideus angeordnet sind.

12. Schlafapnoeerkennungssystem zur Durchführung eines Verfahrens nach den Ansprüchen 1 bis 11, zur Diagnose eines Pharynxverschlusses bei dem obstruktiven Schlafapnoesyndrom, umfassend mindestens ein Elektrode zur Applizierung eines Stroms und/oder zur Messung einer Spannung.

13. Schlafapnoeerkennungssystem nach Anspruch 12, wobei das System zwei räumlich voneinander getrennte Elektroden zur Applizierung eines Stroms und/oder zur Messung einer Spannung umfasst, wobei bevorzugt eine Stromquelle mit einer Frequenz von 25 kHz bis 200 kHz, bevorzugt 50 kHz oder 100 kHz mit mindestens einer Elektrode verbunden ist.

14. Cuffelektrode zur Messung der Bioimpedanz und Stimulation der Zunge, wobei die Elektrode an einer Innen- und Außenseite Elektrodenkontakte aufweist und/oder die Elektrode aus Polyimid oder Silikon besteht.

15. Implantat, umfassend ein Schlafapnoeerkennungssystem nach einem oder mehreren der Ansprüche 12 und 13, wobei das Implantat kontinuierlich und quantitativ einen Rachenverschluss erfasst und durch eine geregelte und selektive Stimulation des Nervus hypoglossus den Tonus der Zunge zu erhöhen.
